# EUROPEAN PATENT APPLICATION

(11) **EP 2 767 241 A1**
(43) Date of publication of application: **20.08.2014**
(21) Application number: 14155568.0
(22) Date of filing: 18.02.2014
(51) Int. Cl.: A61B 17/04

(54) **Knotless swivel anchor with anchor body advanced over anchor tip**

(30) Priority: 19.02.2013 US 201361766249 P; 27.02.2013 US 201361770011 P
(71) Applicant: Arthrex, Inc., Naples, FL 34108-1945 (US)
(72) Inventor: Choinski, Ronald J, Fort Myers Beach, FL Florida 33931 (US)
(74) Representative: Carpmael, Robert Maurice Charles

(57) **Abstract**

A suture anchor (100) construct with an anchor tip (10) that securely engages and locks into a cannulated anchor body (30) (a fixation device such as a cannulated interference screw or plug). The anchor tip includes an eyelet (11) that receives a strand (40) attached to tissue or graft, such that the strand is able to freely slide through the eyelet to correctly position tissue attached to the strand against bone. The anchor tip has an outer diameter smaller than the inner diameter of the anchor body (fixation device) and it retracts into the anchor body as the anchor body is advanced into the bone hole to fixate the strand and the attached tissue to bone.

## Description

### FIELD OF THE INVENTION

The present invention relates to a suture anchor for fixation of tissue to bone.

### BACKGROUND OF THE INVENTION

Devices for knotless fixation of tissue are known in the art. For example, a swivel anchor used to capture a flexible strand (suture, suture tape or suture chain) for surgical tissue repair without requiring suture knots is disclosed and described in U.S. Patent Publication No. 2008/0004659. The implant (swivel anchor or SwiveLock™ C anchor) includes a closed aperture or a forked tip to allow free sliding of a suture strand or capturing of a suture chain or graft. The swivel anchor is secured in a hole in bone by advancing a fixation device (such as a cannulated interference screw or plug) over the body of the implant.

Swivel anchors such as SwiveLock™ C anchors have been used successfully in various methods of knotless tissue fixation, for example, arthroscopic repair techniques which include single-row, double-row, SutureBridge™ and SpeedBridge™ techniques, among others. The SpeedBridge™ technique (developed by Arthrex, Inc.) uses, for example, a threaded swivel anchor (such as disclosed in U.S. Patent Publication No. 2008/0004659) combined with FiberTape® (disclosed in U.S. Patent No. 7,892,256) to create a quick and secure SutureBridge construct with no knots and only two suture passing steps.

In the SpeedBridge™ technique, a swivel anchor, preferably an Arthrex 4.75 or 3.5 mm SwiveLock® C anchor, loaded with one strand of FiberTape®, is inserted into a medial bone socket. A FiberLink™ and Scorpion™ is used to shuttle both FiberTape® tails through the rotator cuff simultaneously. Next, one FiberTape® tail from each medial anchor is retrieved and loaded through the SwiveLock® C eyelet. The loaded eyelet is inserted into a prepared lateral bone socket until the anchor body contacts bone, and the tension is adjusted if necessary. The SwiveLock® C driver is rotated in a clockwise direction to complete the insertion. Using an open ended FiberWire® cutter, the FiberTape® tails are cut, one at a time, to complete the technique. A similar SpeedBridge™ technique is also used to repair the Achilles tendon.

There is a need for improved methods for attaching soft tissue to bone which do not require multiple suture knots and which allow the tendon to remain securely in place until the ligaments naturally attach to bone. Also needed is a method of tissue fixation with minimum drill and anchor depth using swivel anchors (such as SwiveLock® C anchors) in applications that require shallower depth. A method of tissue fixation wherein the eyelet of the SwiveLock® C anchor directs and holds suture in proper orientation is also needed. A new swivel anchor with a design that reduces the overall length of the construct and that also eliminates the wasted drill depth in space-limited applications (for example, small joints such as hand and wrist applications) is also needed.

### SUMMARY OF THE INVENTION

The knotless suture anchor of the present invention includes an anchor tip that securely engages and locks into a anchor body (e.g., a fixation device such as a cannulated interference screw or plug). The anchor tip of the knotless anchor includes an aperture/eyelet for receiving at least one flexible strand attached to tissue or graft, such that the flexible strand is able to freely slide through the aperture. The anchor tip has an outer diameter smaller than the inner diameter of the anchor body (fixation device) and it drops (retracts) into the anchor body as the anchor body is advanced into the bone hole. The anchor body may be provided with a ridge located within the anchor body (about half way down) to stop the anchor tip with eyelet and prevent the anchor tip from coming all the way out of the proximal end of the anchor body.

As the anchor body is inserted into the bone, the anchor body passes over the anchor tip with eyelet to reduce the overall anchor depth so the anchor can be used in space-limited applications such as small joint applications. The cannulated anchor body can be made from PEEK, metal or biocompatible materials.

The knotless anchor of the present invention is designed to seat the anchor tip with eyelet within the anchor body (e.g., fixation device) to minimize the overall length of the construct. This aspect is important as it allows for a shorter bone hole which is particularly important in small joint applications, but also in shoulder and knee repairs as well.

Other features and advantages of the present invention will become apparent from the following description of exemplary embodiments of the invention described with reference to the accompanying drawings.

### BRIEF DESCRIPTION OF THE DRAWINGS

FIG. 1 illustrates an exemplary swivel anchor (with a fixation device and an eyelet) according to the present invention.
FIG. 2 illustrates the swivel anchor of FIG. 1 with the eyelet in the retracted position (fully covered).
FIG. 3(a) illustrates a perspective view of an anchor body of the present invention (a vented 3.5mm swivelock screw provided with a plurality of openings or fenestrations).
FIG. 3(b) illustrates a right side view of the anchor body of FIG. 3(a).
FIG. 3(c) illustrates a front view of the anchor body of FIG. 3(a).
FIG. 3(d) illustrates a cross-sectional view of the anchor body of FIG. 3(c).
FIG. 3(e) illustrates a left side view of the anchor body of FIG. 3(a).
FIG. 3(f) illustrates an enlarged view of detail B of the anchor body of FIG. 3(a).
FIG. 4(a) illustrates a perspective view of an exemplary anchor tip with eyelet (a 2.9mm pushlock anchor tip with eyelet or a 3.5 mm swivel anchor tip with eyelet) of the present invention (closed aperture).
FIG. 4(b) illustrates a left side view of the anchor tip of FIG. 4(a).
FIG. 4(c) illustrates a top view of the anchor tip of FIG. 4(a).
FIG. 4(d) illustrates a cross-sectional view of the anchor tip of FIG. 4(a).
FIG. 4(e) illustrates a right side view of the anchor tip of FIG. 4(a).
FIG. 5(a) illustrates a perspective view of another exemplary anchor tip (forked tip swivel anchor eyelet; swivelock tip extended) of the present invention.
FIG. 5(b) illustrates a right side view of the anchor tip of FIG. 5(a).
FIG. 5(c) illustrates a front view of the anchor tip of FIG. 5(a).
FIG. 5(d) illustrates a cross-sectional view of the anchor tip of FIG. 5(c) taken along line A-A.
FIG. 6(a) illustrates an exemplary assembly of a driver with a swivel anchor with anchor tip and eyelet of the present invention (a 3.5mm swivel anchor in an exemplary length of 10mm).
FIG. 6(b) illustrates another exemplary assembly of a driver with a swivel anchor with anchor tip and eyelet of the present invention (a 3.5mm swivel anchor in an exemplary length of 8mm).
FIG. 7 illustrates the 10mm swivel anchor of FIG. 6(a), with the anchor body extending over the anchor tip and eyelet.
FIG. 8 illustrates a perspective view of an inserter used with the swivel anchor of FIGS. 1 and 2.
FIG. 9 illustrates a prior art SwiveLock® C anchor (with a length of about 15mm).
FIG. 10 illustrates a swivel anchor with anchor tip and eyelet of the present invention (with a length of about 10mm).
FIG. 11 illustrates the swivel anchors with anchor tip and eyelet of the present invention, and inserted in bone sockets.

### DETAILED DESCRIPTION OF THE PREFERRED EMBODIMENTS

The present invention provides a suture anchor for knotless tissue. The suture anchor has an anchor tip that securely engages and locks into an anchor body, for example, a cannulated ribbed body of a fixation device such as a cannulated interference screw or plug. In an exemplary embodiment, the suture anchor is a swivel anchor. The anchor tip of the swivel anchor includes an aperture for receiving a strand attached to tissue or graft, such that the strand is able to freely slide through the aperture. The anchor tip has a diameter smaller than the inner diameter of the anchor body (for example, threaded cannulated screw) and it drops (retracts) into the anchor body as the anchor body is advanced into the bone hole. Preferably, the anchor body is provided with a ridge located within the body of the anchor body (about half way down) to stop the anchor tip with the eyelet and to prevent the anchor tip from passing completely through the anchor body.

As the anchor body (e.g., cannulated interference screw or plug) is inserted into the bone, the anchor body passes over the anchor tip with eyelet to reduce the overall anchor depth so the anchor can be used in space-limited applications such as small joint applications. The cannulated anchor body can be made from PEEK, metal or biocompatible materials.

The suture anchor of the present invention is designed to seat the anchor tip with eyelet within the fixation device to minimize the overall length of the construct. This aspect is important as it allows for a shorter bone hole which is particularly important in small joint applications, but also in shoulder and knee repairs as well.

According to an exemplary embodiment of the invention, and as detailed below, a flexible strand is passed through the soft tissue or graft at desired points. A cannulated screw or plug (of a swivel or pushlock anchor) is pre-loaded onto a driver provided with an anchor tip having a retractable (retracting) eyelet at its distal end. The flexible strand attached to the soft tissue or graft is passed through the aperture of the anchor tip located at the distal end of the driver. The distal end of the driver together with the anchor tip (implant) is inserted directly into the bone. The driver may be rotated (in a clockwise direction, for example) to advance the cannulated screw over the anchor tip and eyelet (i.e., passing over the anchor tip with the retracting eyelet) to complete insertion. Preferably, the cannulated screw is advanced to fully cover about all exterior surface of the anchor tip. The anchor tip is threaded onto the driver using a reverse thread so that, as the driver is rotating to advance the cannulated anchor body, the anchor tip with eyelet is unthreading from the driver so that, once the cannulated anchor body is inserted, the driver can be removed leaving the anchor tip with the eyelet behind.

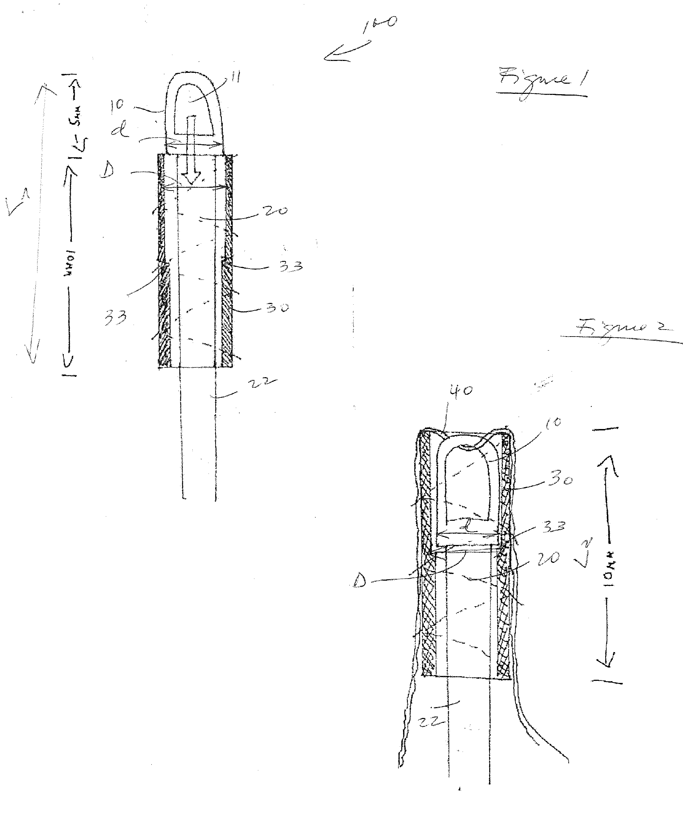

Referring now to the drawings, where like elements are designated by like reference numerals, FIGS. 1 and 2 illustrate an exemplary 10mm swivel anchor 100 (anchor construct 100) with anchor tip 10 with eyelet or aperture 11 formed according to exemplary embodiments of the present invention. Swivel anchor 100 is a knotless fixation device provided with retracting anchor tip 10 attached to driver 20, and anchor body 30 in the form of a fixation device, e.g., a cannulated interference screw or plug that is pre-loaded on the driver 20.

Eyelet 11 is essentially an aperture for receiving a strand 40 attached to soft tissue or graft, such that the strand 40 is able to freely slide through the aperture 11. The aperture may be a closed aperture or a forked tip aperture (open aperture). The retractable anchor tip 10 has an outer diameter "d" smaller than the inner diameter "D" of the fixation device 30.

Anchor body 30 (fixation device 30) is cannulated and provided with a ridge or ledge 33 (preferably of at least 0.25 mm) extending within the inner body of the anchor body 30 (about half way down), to stop the anchor tip 10 and eyelet 11 and to prevent the anchor tip 10 from passing completely through the anchor body, as detailed below. Anchor body 30 may be provided with internal threads (or partial threads) to aid in the advancement of the anchor body relative to the anchor tip 10 and fully over the anchor tip 10, as described below. In an exemplary-only embodiment, anchor body 30 is a fixation device in the form of a cannulated screw, for example, a cannulated interference screw provided with internal threads on at least a portion of its internal cannulation. The anchor body 30 may be threaded (as part of a swivelock anchor) or non-threaded (as part of a pushlock anchor).

A flexible strand 40 (for example, a suture strand or FiberTape® 40) is passed through eyelet 11 of the anchor tip 10 of the knotless fixation device, as shown in FIG. 2.

In the unlocked (unretracted) position shown in FIG. 1, the length L₁ of the swivel anchor is about 15 mm. In the retracted position shown in FIG. 2, however, the length L₂ of the swivel anchor is only about 10 mm, a significant reduction in length which allows for a shorter bone hole which is particularly important in small joint applications (such as hand and wrist applications, for example), but also in shoulder and knee repairs as well. In the retracted position, anchor tip 10 with eyelet 11 is completely surrounded by (housed within) the anchor body 30 (fixation device 30) so that the anchor body covers mostly all external surface of the anchor tip 10, preferably covers all external surface of the anchor tip 10.

As the anchor body 30 (cannulated interference screw 30) is inserted into the bone, the anchor body 30 advances over (passes over) the anchor tip 10 with the captured suture, to reduce anchor depth so the anchor can be used in space-limited applications such as small joint applications. Swivel anchor 100 is designed to fully seat the anchor tip 10 within the anchor body 30 (to fully house the eyelet 10 within the inner cannulation of the anchor body) to minimize the overall length of the construct. This aspect is important as it allows for a shorter bone hole which is particularly important in small joint applications, but also in shoulder and knee repairs as well.

According to an exemplary-only embodiment of the invention, the strand 40 is passed through the graft at desired points. A threaded anchor body 30 (a cannulated screw 30) is pre-loaded onto shaft 22 of driver 20 provided with an anchor tip 10 with eyelet 11 at its distal end. The shaft 22 may have a diameter of about 1.0mm. The strand 40 attached to the graft is passed through the aperture 11 of the anchor tip 10 (implant 10) located at the distal end of the driver shaft 22. The distal end of the driver together with the implant is inserted directly into the bone. The driver 20 is rotated (in a clockwise direction, for example) to advance the anchor body 30 over the anchor tip and the eyelet (i.e., passing over the anchor tip 10), while the anchor tip 10 is held stationary, to complete insertion and form swivel anchor 100.

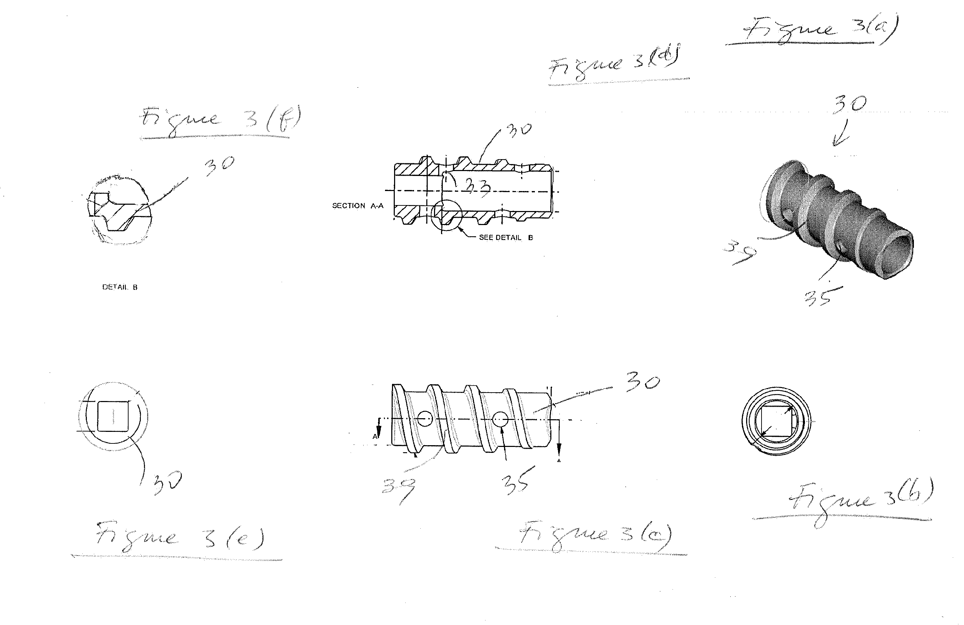

FIGS. 3(a)-3(f) illustrate various detailed views of anchor body 130 (fixation device 130). Fixation device 130 is, for example, a 3.5 mm screw which is fully cannulated and provided with inner ridge 33 and a plurality of vents 35 (openings or fenestrations 35), as shown in FIG. 3(c). The anchor body 130 may be internally threaded or internally non-threaded. As shown in FIG. 3(a), anchor body 130 is fully threaded on the outside surface, i.e., provided with external threads 39 extending from a most proximal end to a most distal end of the anchor body 130.

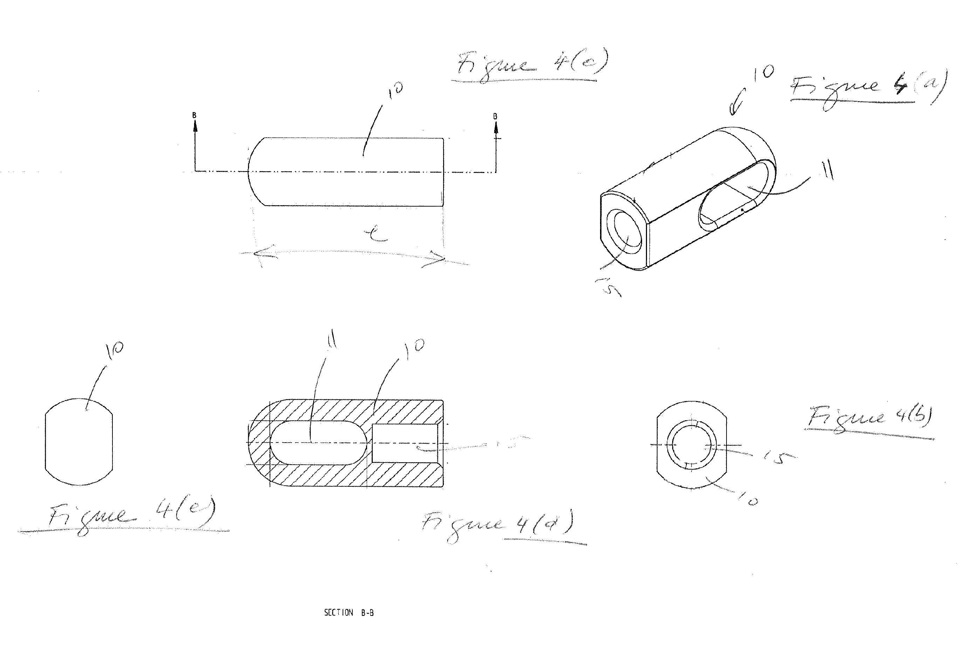

FIGS. 4(a)-4(e) show detailed views of anchor tip 10 with eyelet/aperture 11 of the swivel anchor 100 of the present invention. Anchor tip 10 is also provided with an internal cannulation 15 extending from a most proximal end of the anchor tip 10 and extending within the body of the anchor tip 10 for about a half the length "1" of the anchor tip, as shown in FIGS. 4(c) and 4(d). Internal cannulation 15 aids in removably attaching to and detaching from driver shaft 22. In an exemplary only embodiment, anchor tip 10 is a 3.5mm eyelet formed of PEEK or similar biocompatible materials and provided with closed aperture 11. Any type of flexible material or suture (such as FiberWire®, FiberTape®, FiberChain®, etc.) may be passed through the aperture 11 of the anchor tip 10 to complete various tissue repairs.

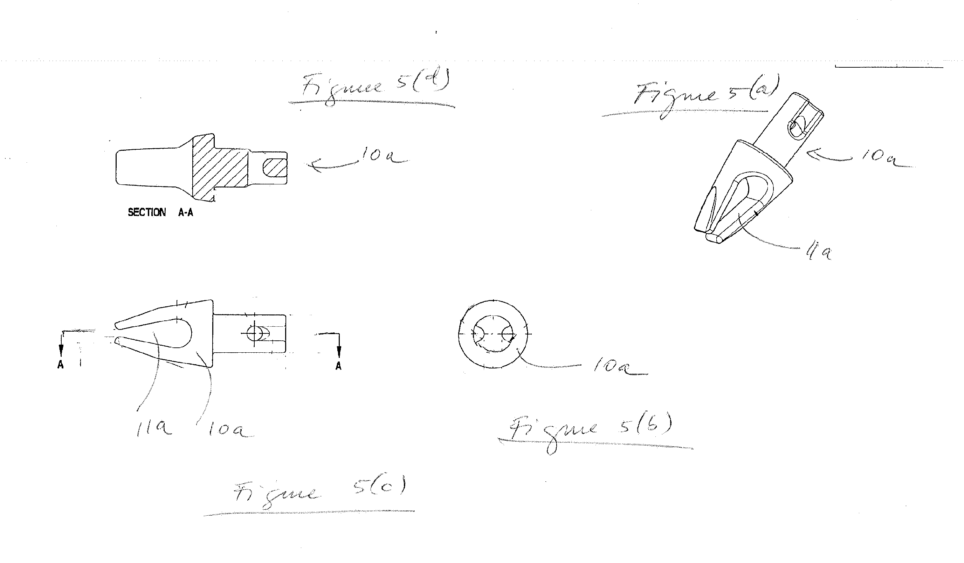

FIGS. 5(a)-5(d) illustrate various views of another anchor tip 10a with open eyelet/aperture 11a of a swivel anchor of the present invention, and according to yet another exemplary embodiment. Open eyelet/aperture 11a of the anchor tip 10a is a forked tip (forked opening) that can capture one or more flexible strands, preferably a suture chain or soft tissue or graft. The open aperture 11a of anchor tip 10a can have many shapes or geometries as long as it is provided open, the embodiment of FIGS. 5(a)-5(d) being only exemplary.

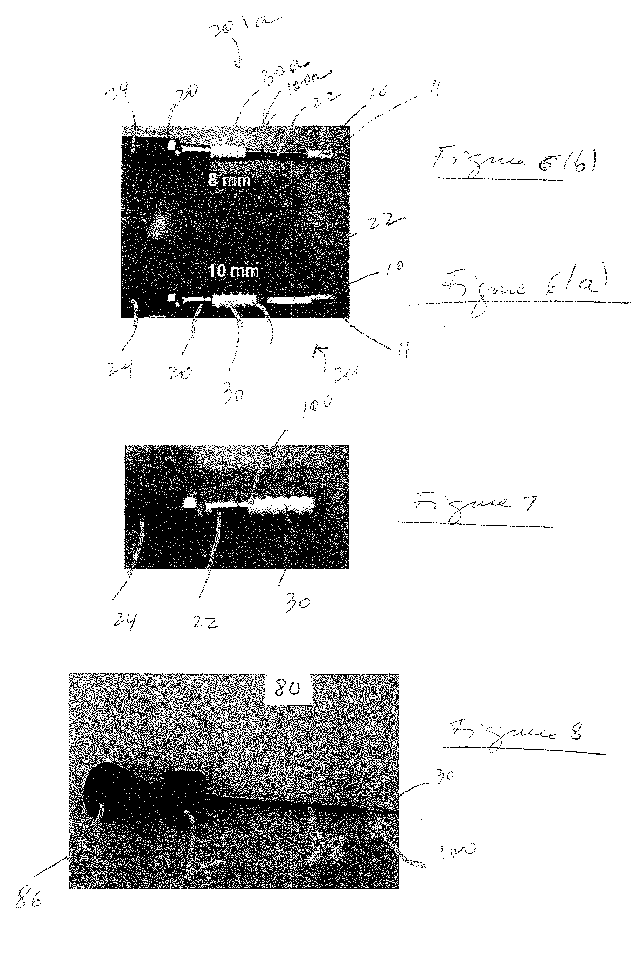

FIGS. 6(a) and 6(b) illustrate two exemplary-only anchor assemblies 201, 201a comprising driver 20 attached to swivel anchor 100, 100a with exemplary anchor tip 10, 10a and eyelet 11, 11a of the present invention. Driver 20 includes shaft 22 that engages anchor tip 10 at one end, and is attached to handle 24 at the other end.

Swivel anchors 100, 100a are exemplary 3.5 mm swivel anchors provided in two different lengths, 8 mm and 10 mm. Swivel anchor 100 of FIG. 6(a) is a 10 mm construct (shown also above in FIGS. 1 and 2), i.e., the length of the anchor body 30 (fixation device) is about 10mm. Swivel anchor 100a of FIG. 6(b) is an 8 mm construct, i.e., the length of the anchor body 30a (fixation device) is about 8 mm.

FIG. 7 illustrates the 10 mm swivel anchor 100 of FIG. 6(a) with anchor tip 10 and eyelet 11 completely covered by the anchor body 30 (fixation device 30). Anchors 100, 100a are designed to fully seat the anchor tip 10, 10a with eyelet 11, 11a within the anchor body 30, 30a, 130 in order to minimize the overall length of the construct (as shown in FIG. 7).

FIG. 8 illustrates a perspective view of an inserter 80 used with the swivel anchor 100, 100a described above. Inserter 80 may be similar to a regular SwiveLock® inserter, as detailed and described in U.S. Patent Publication No. 2008/0004659, the disclosure of which is incorporated by reference in its entirety herewith. To secure at least one flexible strand within a bone tunnel or socket, at least one flexible strand is captured with aperture 11, 11a of the anchor tip 10, 10a. Anchor body 30, 30a, 130 which is already loaded onto the shaft of the inserter is then advanced towards the anchor tip 10, 10a (with the captured flexible strand) by holding the thumb pad 85 as the inserter handle 86 is turned clockwise. In this manner, the anchor body 30, 30a, 130 (fixation device 30, 30a, 130) is inserted into the bone hole or tunnel and positioned behind the anchor tip 10, 10a while the anchor tip is held stationary. As the inserter handle 86 is further turned/rotated and as the anchor tip 10, 10a is held stationary, the anchor body 30, 30a, 130 continues to advance over the anchor tip 10, 10a and to completely cover the anchor tip 10, 10a and the eyelet 11, 11a with the captured flexible strand 40, to fully engage and fully seat the anchor tip 10, 10a and the captured strand into bone 95. Further rotation of the inserter 80 will not result in additional movement of the anchor body 30, 30a, 130 as the anchor tip is stopped by the internal ridge 33 of the anchor body 30, 30a, 130 (fixation device 30, 30a, 130).

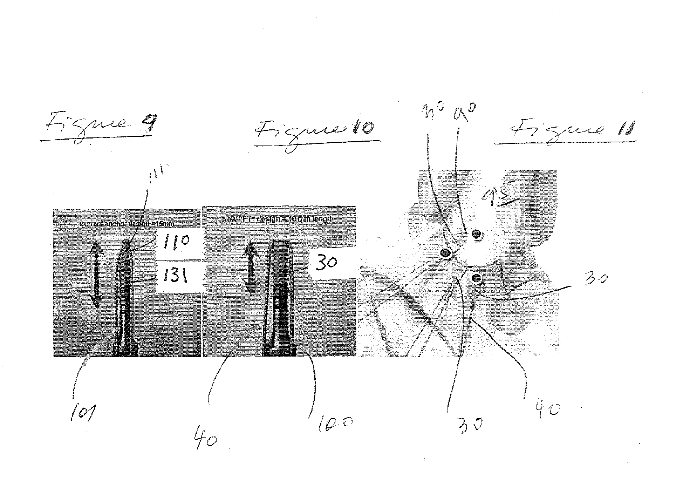

Reference is now made to FIGS. 9-11, which illustrate comparative examples of a prior art SwiveLock® C anchor 101 (FIG. 9) and a swivel anchor 100 of the present invention (FIG. 10), and methods of deploying such anchors into bone (FIG. 11).

FIG. 9 illustrates a SwiveLock® C anchor 101 having a length of about 15 mm (i.e., an extended length). Anchor tip 110 with eyelet 111 of the SwiveLock® C anchor 101 is non-retracting and the design of the construct does not allow anchor body 131 (fixation device 131 in the form of a cannulated interference screw or plug) to advance and fully cover the anchor tip with eyelet 111.

FIG. 10 illustrates swivel anchor 100 of the present invention with anchor tip 10 and eyelet 11 of the present invention fully covered by the anchor body 30 (fixation device 30). The overall length of the construct is significantly decreased to about 10mm (or 8mm) as opposed to the 15 mm of the SwiveLock® C anchor 101 of FIG. 9.

FIG. 11 illustrates swivel anchors 100, 100a with anchor tip 10, 10a and eyelet 11, 11a of the present invention inserted into various bone sockets or holes, during arthroscopic repair techniques which include single-row, double-row, SutureBridge™ and SpeedBridge™ techniques, among many others.

Subsequent to the formation of pilot holes 90 in bone 95, and referring to FIG. 11, a swivel anchor tip 10 with eyelet 11 loaded with at least one strand of suture 40 (or suture tape 40 or any flexible material 40), is placed in the pre-formed hole 90. The inserter 80 (FIG. 8) is then rotated to advance anchor body 30 (cannulated interference screw 30) down shaft 88 to secure the anchor tip and captured suture in the bone hole 90. More specifically, as shown in FIG. 8, the anchor body 30 is advanced by holding thumb pad 85 as the driver handle 86 is turned clockwise. As the swivel anchor driver 80 is rotated in clockwise direction, the anchor body 30 (screw 30) is inserted into the bone to completely pass over the anchor tip 10, and fully cover an external surface of the anchor tip 10. This step may be repeated for other lateral and/or medial bone sockets, to secure additional swivel anchors and/or pushlock anchors, into bone and complete the repair. An exemplary tissue repair which may employ swivel anchors 100, 100a of the present invention is detailed and described, for example, in U.S. Patent No. 8,012,174.

The present invention also provides a lateral anchor repair kit utilizing the unique new anchors 100, 100a of the present invention. Benefits of this design and kit include:
- Knotless (zero profile) fixation of the lateral ligaments;
- Allows tactile hand tensioning of the ligaments;
- Strong twist in fixation improves surgeon control and increases pull out;
- Anchor depth appropriate for the procedure; and
- Multiple FiberWire® or FiberTape® size options are included and can be used according to surgeon's preference, for example, #0, #1, and #2.

This kit employs at least one or multiple 3.5 mm swivel anchors 100, 100a that recess the PEEK anchor tip 10 with eyelet 11 into the anchor body, to allow for full thread purchase and greatly reduce the depth of the device which is too deep for applications. About on-third of anchor depth is needed for the suture eyelet. The new fully threaded design of the present invention eliminates wasted drill depth in space-limited applications.

According to an exemplary-only method, the surgeon tags stitch torn ligament then drills two anchors appropriately placed into the inferior aspect of the fibula. Once the hole is drilled, the sutures are loaded into the anchor placed into the hole and tensioned appropriately. The anchor is deployed and the remaining suture ends with needle can be stitched through the remaining ligament. A third anchor may be placed superiorly, to further reinforce the construct and complete the repair.

Although the embodiments above have been described with reference to a suture anchor with an anchor tip and eyelet that is stationary and an anchor body (fixation device) that moves so that the anchor body advances over (and covers at least partially, preferably fully covers) the anchor tip, the present invention is not limited to these embodiments. Accordingly, the present invention also contemplates embodiments with suture anchors having a non-stationary eyelet, i.e., where the eyelet *itself* retracts into the body of the fixation device (e.g., the cannulated screw).

The 3.5 mm swivel anchors 100, 100a of the present invention have applicability to shoulder repairs (rotator cuff repairs) but also to small joint repairs hand and wrist applications such as scapholunate repair, and to foot and ankle repairs such as lateral ankle, Achilles tendon repair and tendon reattachment (where there are so many instances where the 15 mm length is simply too long and would likely be in a joint space). The anchor tip with eyelet of the implant makes up 1/3 of the anchor length and does nothing for fixation other than hold the suture. By designing the anchor 100, 100a so that the PEEK anchor tip 10 is smaller than the inner diameter of the anchor body 30, 30a, 130, the anchor tip with eyelet is enclosed by into the anchor as it is advanced into the hole. The ridge 33 provided in the anchor half way down stops the anchor tip with eyelet and prevents it from passing completely through the anchor body. This aspect opens up a lot of applications and provides full thread fixation for the entire depth of the drill hole. An exemplary 8mm construct has particular applicability to the hand and wrist applications, as well.

The knotless fixation devices of the present invention advantageously minimize or eliminate the need to tie knots. The use of such swivel anchors also provides secure fixation of the suture construct, the secure suture construct resulting from the suture being pushed into a hole and held tightly by anchors.

In the preferred embodiment of the present invention, as mentioned above, suture or suture tape is used with the swivel anchor to fix tissue to bone. However, the swivel anchors of the present invention can be used with any type of flexible material or suture, for example FiberWire® or FiberTape® or FiberChain®. In another embodiment, an allograft or biological component may be used instead of suture or tape. The allograft or biological component may be comprised of tendon or pericardium, for example, which provides improved tissue repair. In yet additional embodiments, any combination of suture, suture tape, suture chain, and allograft or biological component may be employed, depending on the characteristics of the specific surgical repair and/or as desired.

A method of tissue fixation according to the present invention comprises *inter alia* the steps of: (i) providing a flexible strand 40 such as a suture strand; (ii) securing the flexible strand 40 to the tissue to be fixated; and (iii) anchoring the flexible strand 40 into the bone socket 90 using a swivel implant 100, 100a with an anchor tip 10, 10a with retractable (retracting) eyelet 11, 11a and an anchor body 30, 30a, 130 (a fixation device such as a cannulated interference screw or plug), thereby providing tissue fixation.

Although the present invention has been described in relation to particular embodiments thereof, many other variations and modifications and other uses will become apparent to those skilled in the art. It is preferred, therefore, that the present invention be limited not by the specific disclosure herein.

## Claims

1. A suture anchor comprising:
an anchor body; and
an anchor tip with an aperture configured to capture suture, the anchor tip being rotatably engagable with the anchor body, the anchor body being insertable over the anchor tip to cover the anchor tip and to secure the suture anchor and the captured suture into the bone.

2. The suture anchor of claim 1, wherein the anchor tip has an outer diameter smaller than an inner diameter of the anchor body, to allow the anchor tip to fully retract within the anchor body when the anchor body is inserted over the anchor tip.

3. The suture anchor of claim 1, wherein the aperture is a closed aperture.

4. The suture anchor of claim 1, wherein the anchor tip is a forked tip and the aperture is an open aperture.

5. The suture anchor of claim 1, wherein the anchor tip is a forked tip configured to swivel relative to the anchor body.

6. The suture anchor of claim 1, wherein the anchor body is a push-in type anchor in the form of a cannulated plug.

7. The suture anchor of claim 1, wherein the anchor body is an interference screw.

8. The suture anchor of claim 1, wherein the anchor body is provided with a ridge located around an inner wall of the anchor body, to stop the anchor tip when the anchor body is inserted over the anchor tip.

9. The suture anchor of claim 1, wherein the anchor has a length of about 8 mm to about 10 mm.

10. The suture anchor of claim 1, wherein the anchor tip has a length of about 5 mm.

11. A suture anchor assembly comprising the suture anchor of claim 1, and a driver with a handle and a shaft, wherein the anchor body is pre-loaded on the shaft of the driver and the anchor body is insertable over, and engageable with, the anchor tip, to fully cover the anchor tip and to secure the suture anchor with the captured suture in bone.

12. The suture anchor assembly of claim 11, wherein the shaft is threaded and the anchor body is a cannulated interference screw.

13. The suture anchor assembly of claim 11, wherein the anchor tip is detachable from the shaft.

14. The suture anchor assembly of claim 11, wherein the anchor body is a plug or a cannulated screw provided with an inner ridge or ledge that prevents movement of the anchor tip within the anchor body and along a length of the anchor body, when the anchor body is inserted over the anchor tip.
